# EUROPEAN PATENT SPECIFICATION

(11) **EP 0 550 955 B1**
(45) Date of publication and mention of the grant of the patent: **19.08.1998**
(21) Application number: 92309759.6
(22) Date of filing: 23.10.1992
(51) Int. Cl.: C12N 15/13, C12N 5/10, C12P 21/08

(54) **Chimeric antibody against human GMP-140**
Chimärer Antikörper gegen menschliches GMP-140
Anticorps chimérique contre le GMP-140 humain

(30) Priority: 08.11.1991 JP 319626/91
(43) Date of publication of application: 14.07.1993
(73) Proprietor: TAKARA SHUZO CO. LTD., Fushimi-ku Kyoto 612 (JP)
(72) Inventor: Kurome, Toru, Izumo-shi, Shimane-ken (JP); Hashino, Kimikazu, Takatsuki-shi, Osaka-fu (JP); Kato, Ikunoshin, Uji-shi, Kyoto-fu (JP); Murakami, Keiko, Kyoto-shi, Kyoto-fu (JP); Katayama, Masahiko, Otsu-shi, Shiga-ken (JP)
(74) Representative: Marlow, Nicholas Simon

(56) References cited:
- WO-A-89/11538
- WO-A-90/11676
- JP-A- 3 150 465
- Proc. Natl. Acad. Sci, USA vol.86, pages 4220-4224 (LoBuglio et al) 1989
- J. Nuclear Medicine, vol.32, pages 1162-1168 (Meredith et al) 1991
- Immunity to Cancer, vol.II, pages 101-105 (Marchito et al) 1989, Alan R.Liss, Inc.
- Cancer Imaging with Radio labelled Antibodies, pages 413-423 (Greiner et al) 1990
- J.Immunology, vol.144, pages 3211-3217 (Hoogenboom et al) 1990

## Description

This invention relates to a process for producing mouse/ human chimeric antibody and transformed cells used therefor. The mouse/human chimeric antibody is capable of recognizing human GMP-14Ω.

When a vascular endothelium is damaged in a human body and the subcutaneous tissue thereof is bared, platelets in the flowing blood adhere to the bared site and liberate various platelet stimulants. These substances activate succeeding platelets to cause agglutination thereof with the platelets which have adhered to the affected site, thus forming a thrombus. The formation of the thrombus induces the outbreak of, for example myocardial infarction or cerebral thrombosis and worsens art rial sclerosis. Such a state of the platelets activated as described above may be regarded as pre-thrombosis or thrombophilia. In order to grasp accurately the pathologic conditions of thrombosis, it is highly important from the clinical viewpoint to rapidly diagnose the abovementioned state. From the viewpoint of urgent examination, furthermore, it has been required to establish a method for quickly and conveniently diagnosing the above-mentioned state.

Recently, a glycoprotein of a molecular weight of 140,000 named GMP-140, which appears not on any resting platelet but specifically on the surface of the membrane of an activated platelet, has been identified and attracted public attention as a substance usable as an indication of the extent of the activation of platelets.

Furthermore, attempts have been made to apply monoclonal antibodies against the above-mentioned GMP-140 not only to basic studies, for example, activation of platelets, activation of vascular endothelial cells and mechanism of the formation of inflammation, but also to clinical purposes, for example, serum diagnosis, diagnostic imaging of thrombotic inflammation with the use of a labelled antibody and administration to living organisms of an antibody having antithrombotic and anti-inflammatory effects.

There have been developed various monoclonal antibodies against the GMP-140, since R. P. McEver et al. reported a clone S12 [refer to J. Biol. Chem., 259, 9799 (1984)] and B. Furie et al. reported another clone KC4 [refer to J. Biol. Chem., 259, 9121 (1984)].

The present inventors also successfully established strains (FERM P-11072 and FERM P-11073) capable of producing anti-human GMP-140 monoclonal antibodies (WGA-1 and PL7-6) suitable for assaying the GMP-140 [refer to Japanese Patent Laid-Open No. 150465/1991] and clarified the relationship of the GMP-140 with diseases by assaying free GMP-140-like substances present in blood samples with the use of these antibodies [refer to Japanese Patent Laid-Open No. 22866/1992].

However these anti-GMP-140 monoclonal antibodies are ones derived from mouse hybridomas. Therefore, the application of these mouse antibodies to clinical purposes, for example, human drugs or diagnostic imaging, is restricted by the immunogenicity of these antibodies. That is, the use of the mouse antibody often induces an immune response to thereby cause anaphylactic shock and the repeated administration thereof results in a decrease in the effects thereof or extremely rapid clearance thereof from the blood, which substantially deteriorates the therapeutic value of the antibody. Although these problems accompanying the application of the above-mentioned mouse antibody can be overcome by using a human monoclonal antibody for clinical purposes, it is greatly difficult to stably produce a human monoclonal antibody against human GMP-140 while maintaining the desired specificity and affinity.

It is known that a strain capable of producing a monoclonal antibody generally suffers from a decrease in the productivity of the antibody as the strain is subcultured. In order to solve this problem, it is required to examine the possibility of expression on a large scale through cell cloning of antibody-producing cells or cloning of antibody genes followed by gene introduction.

Under these circumstances, it may be concluded that a chimeric antibody, which has been constructed by the recombinant DNA technology and consists of a variable region derived from an antibody of one species and a constant region derived from an antibody of another species, commonly a variable region of a mouse antibody and a constant region of a human antibody, has substantially a lower immunogenicity than that of the mouse antibody and thus is more suitable for clinical applications. Chimeric antibodies have been reported by, for example, Shaw et al. in J. Immunol., 138, 4534 (1987) and Morrison, S.L., et al. in Proc. Natl. Acad. Sci. USA, 81, 6851-6855 (1984) and now are regarded as a common idea.

In PCT International Publication No. WO89/11538, a chimeric antibody which specifically recognizes complex of gpIIb/IIIa receptor is described. However, the antibody is not capable of recognizing GMP-140. Up to the present, the amino acid sequences and the gene structure of H and L chain of monoclonal antibodies to GMP-140 such as PL7-6 are not known. The amino acid sequences and gene structure of the variable region of above-mentioned antibodies which specifically recognizes and binds to the antigen are not known. Moreover, it is not known whether the objective anti-GMP-140 antibodies can be produced by the method of genetic engineering, and whether the antibodies have the activity of recognizing the antigen.

A mouse/human chimeric antibody, which can recognize human GMP-140 is highly safe and useful in diagnostic imaging and antibody drugs.

The present invention may be summarized as follows. The first aspect of the present invention relates to an isolated DNA fragment encoding a mouse antibody variable region of a chimeric antibody which binds human GMP-140 specifically and as claimed in claim 1.

The second aspect of the present invention relates to a method for producing a chimeric antibody which binds human GMP-140 specifically comprising the following steps;
a) transforming mammalian cells with a vector containing a mouse H chain variable region DNA fragment of the first aspect of the invention and a human H chain constant region DNA fragment, and another vector, containing a mouse L chain variable region DNA fragment of the first aspect of the invention and a human L chain constant region DNA fragment.
b) isolating the chimeric antibody from the suitable culture medium of the transformed cells.

The third aspect of the present invention relates to a transformed cell which is transformed with a vector containing a mouse H chain variable region DNA fragment of the first aspect and a human H chain constant region DNA fragment, and another vector containing a mouse L chain variable region DNA fragment of the first aspect and a human L chain constant region DNA fragment, and is capable of producing a chimeric antibody which binds human GMP-140 specifically.

A DNA fragment, or gene, coding for the H chain variable region of a mouse anti-GMP-140 antibody may be prepared by, for example, the following method.

As the cells capable of producing an anti-GNP-140 antibody, for example, the hybridoma PL7-6 (FERM P-11073) described in the above-mentioned Japanese Patent Laid-Open No. 150465/1991 are cultured and a genomic DNA is prepared from these cells. The obtained genomic DNA is partially digested with the restriction enzyme EcoRI so as to give a DNA fragment of approximately 10 to 20 kbp. Then these fragments are cloned into, for example, a phage vector EMBL-4. Thus a genomic DNA library can be prepared.

The target gene may be screened from the genomic DNA library by using the 1.9kbp EcoRI-BamHI fragment of a plasmid pSV2HG1Vpc [refer to FEBS Letters, 244, 301-306 (1989)] as a probe.

As described in the above-mentioned reference, the plasmid pSV2HG1Vpc includes a DNA sequence coding for the H chain variable region of mouse anti-phosphorylcholine antibody and another DNA sequence coding for human IgG γ-H chain constant region. Therefore DNA coding for the H chain J region of the mouse antibody and the enhancer region of the mouse antibody gene are included in the EcoRI-BamHI fragment.

Alternately, the target gene may be screened by analyzing the DNA sequence which codes for the H chain variable region of an anti-human GMP-140 monoclonal antibody PL7-6 and then using a probe which contains the DNA sequence.

A gene coding for the H chain variable region of the anti-human GMP-140 monoclonal antibody PL7-6 is located on EcoRI fragment of approximately 3.2 kbp. The present inventors named this DNA fragment PLH.

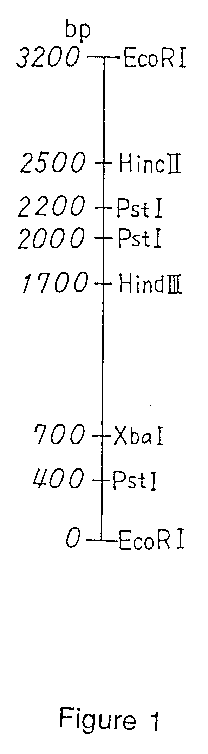

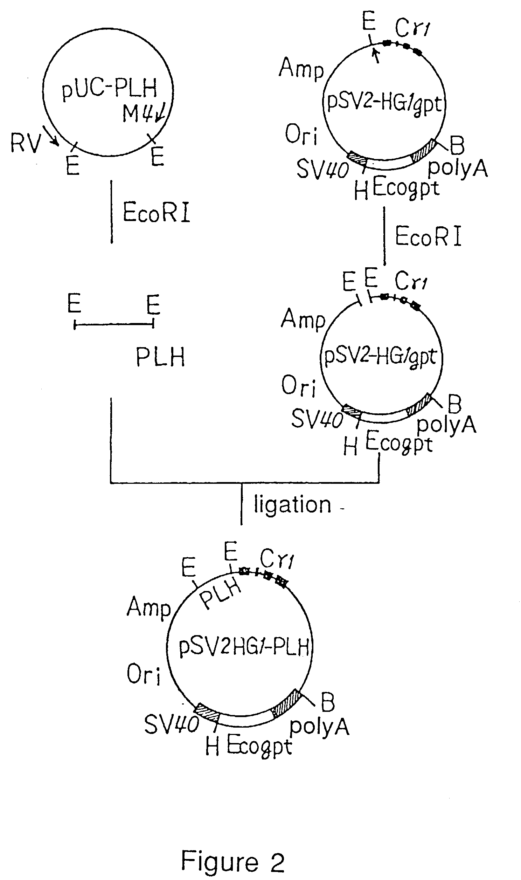

Figure 1 shows a restriction enzyme map of PLH. An expression vector of the target chimeric antibody H chain can be obtained by integrating this PLH, or a DNA fragment prepared by removing unnecessary sequences therefrom, together with an appropriate gene coding for the human H chain constant region into a suitable vector. For example, PLH is inserted into a plasmid pSV2-HGlgpt, which is prepared by removing a DNA coding for the H chain variable region of the mouse anti-phosphorylcholine antibody from the above-mentioned plasmid pSV2HG1Vpc, to thereby prepare a plasmid pSV2HG1-PLH carrying a gene for the H chain variable region of the anti-GMP-140 monoclonal antibody and another gene of human IgG γ-H chain constant region. Figure 2 shows the process for constructing this plasmid as well as the structure thereof, wherein E represents EcoRI while B represents BamHI.

On the other hand, a DNA fragment, or gene, coding for the L chain variable region of the mouse anti-GMP-140 antibody can be screened from the above-mentioned genomic DNA library by analyzing the DNA sequence which codes for the L chain variable region of an anti-human GMP-140 monoclonal antibody PL7-6 and then using a probe which contains the DNA sequence.

The gene coding for the L chain variable region of the anti-human GMP-140 monoclonal antibody is located on an EcoRI-Hind III fragment of approximately 4 kbp which was named PLL by the present inventors.

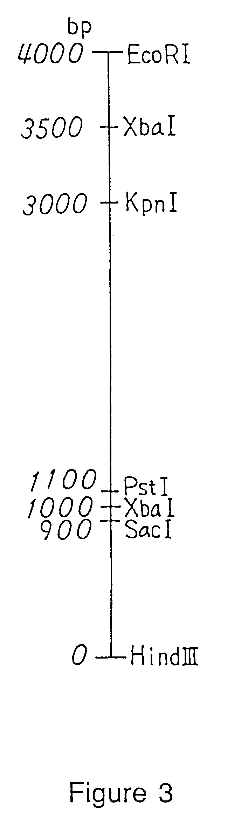

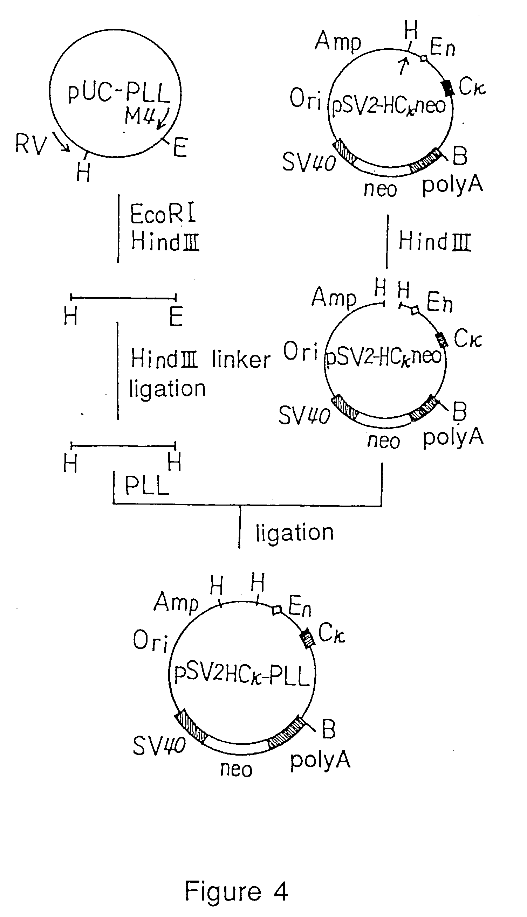

Figure 3 shows a restriction enzyme map of PLL. An expression vector of the target chimeric antibody L chain can be obtained by integrating this PLL, or a DNA fragment prepared by removing unnecessary sequences therefrom, together with an appropriate gene coding for the human L chain constant region into a suitable vector. For example, PLL is inserted into a plasmid pSV2-HCκneo, which is prepared by removing a DNA coding for the L chain variable region of the mouse anti-phosphorylcholine antibody from the plasmid pSV2HCκVpc having a DNA sequence coding for the L chain variable region of the mouse anti-phosphorylcholine antibody and another DNA sequence coding for the human IgG κ-L chain constant region (refer to the above-mentioned FEBS Letters), to thereby prepare a plasmid pSV2HCκ-PLL carrying a gene for the L chain variable region of the anti-GMP-140 monoclonal antibody and another gene for the human IgG κ-L chain constant region. Figure 4 shows the process for constructing this plasmid as well as the structure thereof, wherein H represents Hind III.

The target chimeric antibody can be obtained by transforming appropriate host cells, for example, mouse myeloma SP2/0, with the plasmids pSV2HG1-PLH and pSV2HCκ-PLL and then culturing the transformant thus obtained. The myeloma SP2/0 transformant is named Myeloma SP2-cPL-2R1 and deposited with Fermentation Research Institute of the Agency of Industrial Science and Technology under the accession number FERM BP-3609.

An antibody produced by the transformant is purified by, for example, ion exchange chromatography or affinity chromatography, if required.

The molecule of the present chimeric antibody can be confirmed to have mouse immunoglobulin-like and human immunoglobulin-like structures, for example, in the western blotting analysis. Therefore, according to the standard immunological technology, the chimeric antibody is separated on a polyacrylamide gel electrophoresis, transblotted onto the nitrocellulose membrane, and the proteins can be visualized on the membrane with immunoenzymatic reaction using peroxidase-labelled anti-human IgG antiserum and substrates. The results of this immunochemical analysis can demonstrate that the chimeric antibody has a human immunoglobulin-like constant region.

The antigen specificities of the chimeric antibody of this invention can be confirmed, for example, in another western blotting analysis. Therefore, according to the standard immunochemical technology, lysed platelets are separated on a polyacrylamide gel electrophoresis, transblotted onto the nitrocellulose membrane, and the transblotted proteins can be visualized by peroxidase-linked anti-human IgG antibody and substrates. The platelet antigen recognized by the chimeric antibody can be characterized roughly to analyse the molecular mass of the immunoreactive protein on the membrane.

The antigen-binding affinity of the chimeric antibody can be confirmed in the competitive enzyme-linked immunosorbent assay. Therefore, this assay is performed by putting the chimeric antibody into competition with peroxidase-labelled PL7-6 antibody for binding to the immobilized antigen, and the antigen-binding affinity of the chimeric antibody can be measured to study the percent of its inhibition.

The reactivities of the chimeric antibody with activated platelets can be confirmed by flowcytometric analysis. Therefore, washed platelets are stimulated by thrombin at several concentrations. Thrombin-stimulated platelets are fixed with appropriate reagents and incubated with fluorescence-labelled chimeric antibody. Its reactivity can be confirmed by measuring fluorescence intensity on the surface of these activated platelets in the flowcytometor.

Using the present invention which has been described in detail above, a mouse/human chimeric antibody capable of recognizing human GMP-140 highly useful for clinical purposes can be obtained.

The chimeric antibody can provide fragments capable of specifically recognizing human GMP-140 molecules, such as monovalent Fab fragments or bivalent F(ab')₂ fragments, bispecific chimeric antibodies, single-chain antibodies, enzymes, fluorescent markers, metal chelates, cytostatic substances or cytotoxic substances, conjugates with, for example, avidin, biotin, antiplatelets or thrombolytic agents, and radioisotope-labeled antibodies each by the known method and these products may be used depending on the purpose.

Figure 1 is a restriction map of PLH. Figure 2 is a diagram of the procedure used to construct pSV2HG1-PLH. Figure 3 is a restriction map of PLL. Figure 4 is a diagram of the procedure used to construct pSV2HGκ-PLL. Figure 5 is a diagram of the procedure used to construct pUC-1.9K. Figure 6 is a diagram of the procedure used to construct pUC-PLV_{H}. Figure 7 is a diagram of the procedure used to construct pUC-PLV_{L}. Figure 8 is a graph showing characterization of the antibody by competitive enzyme-linked immunosorbent assay. Figure 9 is a graph showing flowcytometric analysis of the antibody.

### [Examples]

To further illustrate the present invention in greater detail, and not by way of limitation, the following Examples will be given.

### Example 1

### (1) Isolation of genomic DNA from hybridoma PL7-6 cell

A genomic DNA was isolated from hybridoma PL7-6 cells (FERM P-11072) in accordance substantially with a method reported by Pellecer et al. [refer to Cell, 41, 133 (1978)].

More precisely, the above-mentioned cells were cultured and centrifuged at 1,000 rpm for 30 seconds to thereby give approximately 1 x 10⁸ cells, which were suspended in 3 ml of 10 mM Tris-HCl (pH 8.0)-10 mM EDTA-100 mM NaCl-0.2 mg/ml proteinase K under ice-cooling. 150 µl of 10% SDS was added thereto and the sample was incubated at 37°C overnight.

To this reaction mixture was added the same volume of a TE buffer solution (10 mM Tris-HCl, pH 8.0, 0.1 mM EDTA)saturated phenol. The obtained mixture was gently mixed by inversion for 10 minutes and then centrifuged at 10,000 rpm for 10 minutes. This phenol extraction was repeated thrice and the finally obtained supernatant was dialyzed against 3 l of the TE buffer solution.

3 ml of the dialyzed sample was recovered and 15 µl of 0.4 mg/ml RNaseA was added thereto, followed by incubation at 37°C for 1 hour. Next, 30 µl of 20 mg/ml proteinase K and 150 µl of 10% SDS were added thereto, followed by incubation at 37°C overnight.

To the obtained reaction mixture was added the same volume of the TE buffer solution-saturated phenol. After gently mixing by inversion for 10 minutes, the mixture was centrifuged at 10,000 rpm for 10 minutes and the supernatant was recovered. This procedure was further repeated twice except that the same volume of chloroform was used. After adding 2.5 times by volume as much ethanol, the mixture was cooled at -80°C for 30 minutes and then centrifuged at 4°C at 10,000 rpm for 10 minutes. Then the supernatant was removed and the precipitate was washed with 80% ethanol and dried at room temperature under reduced pressure. The obtained precipitate was finally dissolved in 1 ml of the TE buffer solution. Thus PL7-6 genomic DNA was obtained.

### (2) Construction of genomic DNA library relating to PL7-6

15 µg of the PL7-6 genomic DNA was digested in 300 µl of a reaction mixture containing H buffer solution for restriction enzyme reaction (50 mM Tris-HCl, pH 7.5, 10 mM MgCl₂, 1 mM dithiothreitol, 10 mM NaCl) at 37°C with the use of 10 units of EcoRI. Namely, after 5µl portions of 500 mM EDTA were previously pipetted into tubes, 50µl portions of the reaction mixture were sampled and mixed with these EDTA 20, 30, 45, 60, 90 and 120 minutes after the initiation of the reaction to thereby partially digest the genomic DNA. After the completion of the reaction, 8 µl of each sample was subjected to electrophoresis on a 0.8% agarose gel so as to confirm the partial digestion. The remaining samples were mixed together and subjected to electrophoresis on a 0.8% agarose gel. A slice of agarose containing fragments of 10 to 20 kbp was cut out from the gel with the guidance of a molecular marker and subjected to electric elution. Namely, an electric current (100 V, 60 mA) was applied to the gel in a dialysis tube for 70 minutes and the target fragment was recovered from the gel. After removing proteins by effecting the above-mentioned phenol extraction, the residue was precipitated with ethanol. Thus 1.8 µg of the genomic DNA fragment was obtained in 20 µl of the TE buffer solution.

The EMBL-4 phage library was constructed by using EMBL-4 Cloning Kit (Stratagene) in accordance with the kit protocol. Namely, 0.9 µg of the PL7-6 genomic DNA fragment (10 to 20 kbp) and 2 µg of EMBL-4-phage arm were reacted in ligase buffer solution (50 mM Tris-HCl, pH 7.5, 7 mM MgCl₂, 1 mM dithiothreitol) at 4°C overnight with the use of 250 units of T4 DNA ligase.

Packaging was performed by incubating host cells of E. coli strain P2.392, Gigapack II Gold Packaging Mix and 4 µl of the ligated DNA at 22°C for 2 hours in accordance with the protocol of the Gigapack II Gold (Stratagene).

After the completion of the reaction, 500 µl of the SM buffer solution (5.8 g/l NaCl, 2.0 g/l MgSO₄, 50 mM Tris-HCl, pH 7.5) was added thereto. 0.1 µl of the reaction mixture, was sampled to determine the titration of the phage. Next, the mixture was incubated at 37°C for 8 hours with the use of the P2.392 strain. Thus a phage library of 800,000 plaques on NZY plates (5 g/l NaCl, 2 g/l MgSO₄, 5 g/l yeast extract, 10 g/l NZ amine, 15 g/l agar) was prepared.

### (3) Identification and isolation of recombinant phage φE4PLH involving gene coding for PL7-6 mouse antibody H chain variable region

A recombinant phage was screened from the PL7-6 genomic DNA library prepared above by preparing and using two types of DNA probes substantially according to a method described in Experimental Medicine (Jikken Igaku), 5(11) 46 (1987).

### A) Preparation of J_{H}-1.9K probe

15 µg of the above-mentioned plasmid pSV2HG1Vpc described in FEBS Letters was digested with 100 units of EcoRI and BamHI in 100 µl of a reaction mixture containing the H buffer solution for restriction enzyme reaction at 37°C overnight. After subjecting to electrophoresis on a 0.8% agarose gel, a DNA fragment (approximately 1.9 kbp) involving the mouse antibody H chain J region and the enhancer region was recovered from the gel, purified and dissolved in 20 µl of the TE buffer solution.

Next, 4 µg of the plasmid pUC119 was digested with 15 units of EcoRI and BamHI in 50 µl of a reaction mixture containing the H buffer solution for restriction enzyme reaction at 37°C overnight. Then 50 µl of the TE buffer solution was added to the reaction mixture so as to adjust the total volume to 100 µl. Next, 1 units of an alkaline phosphatase originating from E. coli was added thereto and the mixture was reacted at 65°C for 1 hour. After the completion of the reaction, proteins were removed from the reaction mixture by extracting with phenol and the DNA was precipitated with ethanol. The obtained precipitate was dissolved in 50 µl of the TE buffer solution.

The above-mentioned DNA fragment of 1.9 kbp cut from the plasmid pSV2HG1Vpc was ligated into the plasmid pUC119, which had been thus digested with EcoRI and BamHI and dephosphorylated with the use of a Ligation Kit (Takara Shuzo). Thus a plasmid in which a DNA of 1.9 kbp had been integrated was obtained. This plasmid was named pUC-1.9K.

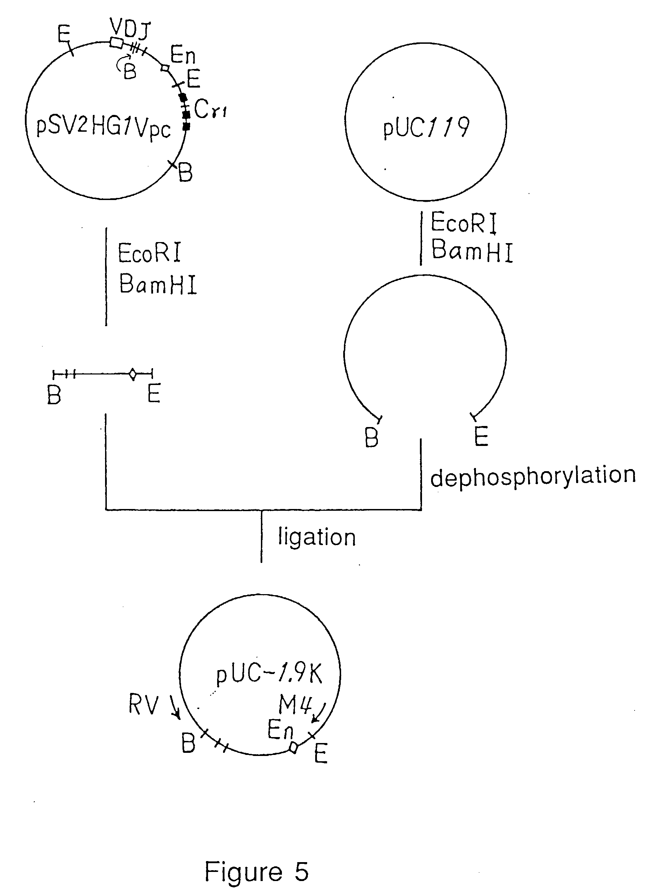

Figure 5 shows a process for constructing this pUC-1.9K, wherein En represents an enhancer.

Next, the DNA fragment of 1.9 kbp in the plasmid pUC-1.9K was amplified by the PCR method. More precisely, 2 µl (4 ng) of the pUC-1.9K constructed above was introduced into a 0.5 ml tube. Then 10 µl of a 10-fold concentrated buffer solution for amplification [100 mM Tris-HCl, pH 8.3, 500 mM KCl, 15 mM MgCl₂, 0.01% (w/v) gelatin] included in a GeneAmp™ Kit (Takara Shuzo), 16 µl of a 1.25 mM dNTP mixture (dATP, dGTP, dCTP, TTP), 0.1 µg (20 pmol) of M4 primer (Takara Shuzo), 0.1 µl (20 pmol) of RV primer (Takara Shuzo) and 0.5 µl of 5 units/µl AmpliTaq™ (Takara Shuzo) were added thereto and the total volume of the mixture was adjusted to 100 µl with sterilized water. After addition 100 µl of Mineral Oil (Sigma) thereon, the fragment was amplified on an automatic amplifier Thermal Cycler (Takara Shuzo). The amplification was effected by repeating a cycle [at 94°C for 30 seconds (thermal denaturation), at 55°C for 2 minutes (annealing) and then at 72°C for 1 minute (synthesis)] 30 times.

Thus approximately 5 µg of a DNA fragment was obtained from 100 µl of the reaction mixture. This fragment was named J_{H}-1.9K probe and used in the subsequent procedures.

### B) Preparation of probe for variable region of H-chain

Hybridoma PL7-6 (FERM P-11073) was cultivated and RNA was prepared from the cell. And then, mRNA was prepared from the RNA by the use of Oligotex™-dT30 (Takara Shuzo). Next, cDNA was synthesized from above-mentioned mRNA by use of reverse transcriptase and primer C-3, specific for constant region of H chain, shown by SEQ ID No. 11 in the Sequence Listing. And then, DNA coding for variable region of H chain was obtained by carrying out PCR with the primer C-3 and mixed primer VH shown by SEQ ID No. 12 in the Sequence Listing. Southern blot analysis of the amplified DNA was performed with probe HP shown by SEQ ID No. 13 in the Sequence Listing. Thus it was confirmed that target DNA was amplified. The amplified DNA was subjected to electrophoresis on agarose gel and then purified by eluting from the gel. And then, the DNA was subcloned into the HincII site of pUC18 after blunting its ends of the DNA with DNA Blunting kit (Takara Shuzo). DNA sequence of the subcloned DNA was analyzed by the dideoxy method. The DNA sequence was shown by SEQ ID No. 1 in the Sequence Listing.

Then 10 µg of this plasmid was digested with 20 units of PstI in 35 µl of a reaction mixture containing the H buffer solution for restriction enzyme reaction at 37°C overnight. Then it was subjected to electrophoresis on a 2% agarose gel, and a fragment of 2.9 kbp was recovered from the gel, purified and dissolved in 100 µl of the TE buffer solution.

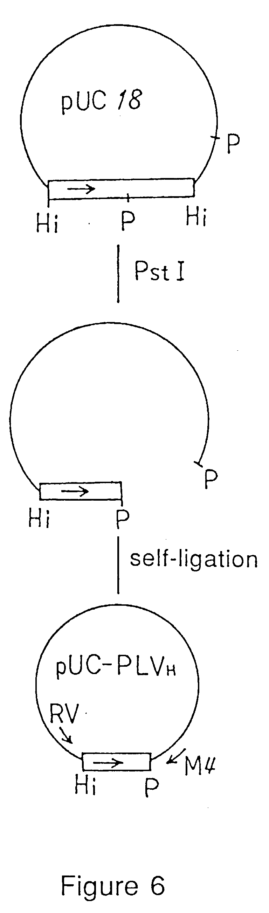

5 µl of the sample solution (100 µl in total) was subjected to self ligation with the use of a Ligation Kit (Takara Shuzo) at 16°C overnight. Thus a plasmid, in which a DNA fragment (216 bp) shown by SEQ ID No. 2 in the Sequence Listing was integrated, was obtained. This plasmid was named pUC-PLV_{H}. Figure 6 shows a process for constructing this plasmid, wherein Hi represents HincII, while P represents PstI.

Next, a DNA fragment shown by SEQ ID No. 3 in the Sequence Listing, which includes the above-mentioned DNA fragment (216 bp) of the pUC-PLV_{H}, was amplified by the PCR method in accordance with Example 1-(3)-A) to obtain approximately 1 µg of a DNA fragment from 100 µl of the PCR reaction mixture. This fragment was named PL-V_{H} probe for use in the subsequent procedures.

### C) Screening

In the primary screening, the above-mentioned PL7-6 genomic DNA library and the probe J_{H}-1.9K was subjected to plaque hybridization. The probe was subjected to non-radioactive labeling with the use of a Chemi Probe Kit (Takara Shuzo) in accordance with the protocol of the kit. In order to eliminate pseudopositive signals, two membranes, which were the same with each other, were taken and the phage clone of which positive signals common to both of these membranes were detected, was selected. Namely, two filters, which had been prepared by the use of nylon membranes Hybond-N (Amersham) according substantially to a method described in Experimental Medicine (Jikken Igaku), 5(11), 46-52 (1987), were immersed in a solution containing 100 µg/ml thermally denatured salmon DNA, 6 x SSC, 5 x Denhardt's solution and 0.1% SDS and incubated at 68°C for 3 hours (prehybridization) . Then 50 ng/ml of the thermally denatured above-mentioned probe J_{H}-1.9K, which had been labeled with the use of a Chemi Probe Kit in accordance with the protocol of the kit, was added to the same solution and incubated at 68°C overnight (hybridization). The membranes were washed twice in a solution containing 2 x SSC and 0.1% SDS at 50°C for 15 minutes each time and then further twice in a solution containing 0.1 x SSC and 0.1% SDS at 50°C for 15 minutes each time. The washed membranes were immersed in a blocking solution prepared in accordance with the indication given in the Chemi Probe Kit and incubated at room temperature for 30 minutes. Next, they were incubated at 37°C for 30 minutes together with a mouse anti-sulfonated DNA monoclonal antibody (a reagent contained in the Chemi Probe Kit) diluted 1000-fold with a conjugate buffer solution (a reagent contained in the Chemi Probe Kit). The membranes thus treated were washed with a washing solution containing 1 x PBS and 0.3% Tween 20 thrice for 10 minutes each time and then incubated at 37°C for 30 minutes together with an alkaline phosphatase-labeled anti-mouse IgG antibody (a reagent contained in the Chemi Probe Kit) diluted 2,500-fold with the conjugate buffer solution. The membranes thus treated were washed with the washing solution thrice for 10 minutes each time and further washed in an assay buffer solution (50 mM NaHCO₃/Na₂CO₃, 1 mM MgCl₂, pH 9.5) twice for 5 minutes each time. After removing the moisture in the membranes, AMPPD (a reagent contained in the Chemi Probe Kit) adjusted to a concentration of 100 µg/ml with the assay buffer solution was added to the membranes and allowed to stand at room temperature for 20 minutes. Then the membranes were introduced into a plastic bag and exposed to light for 20 minutes via laminated XAR Films (Kodak) which the membranes were placed on. Thus 12 positive signals were obtained.

Subsequently, the secondary screening was carried out in the following manner. Namely, the parts of the agar containing the above-mentioned positive clones were taken out of the phage library plate. After being diluted with the SM buffer solution, the phages were inoculated again into an NZY plate and the same procedure as the one performed in the above-mentioned primary screening was effected. Thus a phage clone capable of hybridizing with the probe PL-V_{H} was selected.

By using this recombinant phage clone, a phage lysate was prepared according substantially to a method described in Experimental Medicine (Jikken Igaku), 5(11), 28-32 (1987) to thereby give a phage DNA. 5 µg of this phage DNA was completely digested by incubating with the use of 5 units of EcoRI and 0.25 µg of RNaseA in 10 µl of the H buffer solution for restriction enzyme reaction at 37°C for 3 hours. The obtained reaction mixture was subjected to 1% agarose gel electrophoresis and then transferred onto the nylon membranes Hybond-N (Amersham) by the conventional method. Thus the phage DNA underwent Southern hybridization with the probes J_{H}-1.9K and PL-V_{H} respectively. Thus a DNA fragment of approximately 3.2 kbp capable of hybridizing with both of these probes was detected. The phage clone in which this DNA fragment of approximately 3.2 kbp had been integrated was named φE4PLH.

### (4) Subcloning of phage DNA fragment involving PL7-6 mouse antibody H chain variable region gene

The above-mentioned φE4PLH DNA was isolated and approximately 5 µg thereof was completely digested by incubating with the use of 10 units of EcoRI and 0.5 µg of RNaseA in a reaction mixture containing the H buffer solution for restriction enzyme reaction at 37°C for 3 hours. After being subjected to electrophoresis on a 0.7% agarose gel, a slice of agarose containing a fragment of approximately 3.2 kbp was cut out from the gel and the fragment was purified. Then it was subcloned into the EcoRI site of the plasmid pUC119.

More precisely, 7.6 µg of pUC119 was digested with 15 units of EcoRI in 50 µl of a reaction mixture containing the H buffer solution for restriction enzyme reaction at 37°C overnight. Then 1 units of an alkaline phosphatase originating from E. coli was added to this digested reaction mixture and reacted at 65°C for 1 hour.

To the reaction mixture was added the same volume of the TE-saturated phenol, followed by stirring. Then the mixture was centrifuged at room temperature at 12,000 rpm for 5 minutes to thereby give a supernatant (aqueous phase). To this supernatant was added the same volume of a phenol/chloroform mixture, prepared by mixing 1 part by volume of the TE-saturated phenol with one part by volume of chloroform, and the mixture was stirred. Then it was centrifuged at room temperature at 12,000 rpm for 5 minutes to thereby give a supernatant. To this supernatant was added the same volume of chloroform and the mixture was stirred. Then it was centrifuged at room temperature at 12,000 rpm for 5 minutes to thereby give a supernatant, from which a DNA fragment was recovered by precipitation with ethanol and dissolved in 50 µl of the TE buffer solution. This pUC119, digested with EcoRI and dephosphorylated, was subjected to a ligation reaction with the above-mentioned DNA fragment of approximately 3.2 kbp involving a gene coding for the PL7-6 H chain variable region at 16°C overnight with the use of a Ligation Kit (Takara Shuzo) to thereby give a plasmid in which a DNA of 3.2 kbp had been integrated.

Then E. coli JM109 was transformed by using this plasmid. The obtained transformant was applied onto an L-agar medium plate containing 100 µg/ml of ampicillin (1% trypton, 0.5% yeast extract, 0.5% NaCl, 1.5% agar). Then colonies grown on the plate were cultured in 1.5 ml of an L-broth containing 100 µg/ml of ampicillin (1% trypton, 0.5% yeast extract, 0.5% NaCl). After the completion of the culture, a plasmid was extracted from the cells and dissolved in 100 µl of the TE buffer solution. 2 µl of this plasmid was digested with 10 units of EcoRI in 10 µl of a reaction mixture containing the H buffer solution for enzyme reaction at 37°C for 2 hours.

The reaction mixture was subjected to electrophoresis on a 1% agarose gel and thus a band of approximately 3.2 kbp was confirmed. Simultaneously, it was confirmed with Southern hybridization with the probe J_{H}-1.9K that the DNA fragment of 3.2 kbp inserted into pUC119 was the target one. The DNA fragment of 3.2 kbp was named PLH and the restriction enzyme map of this DNA fragment was prepared. Figure 1 is the restriction enzyme map thus prepared. This fragment carried no recognition site of restriction enzymes SalI, SphI, AccI, ScaI, KpnI, BamHI or SmaI. The plasmid in which this PLH had been integrated was named pUC-PLH.

Next, the E. coli JM109 transformed with this pUC-PLH or pUC-1.9K were each cultured in the L-broth containing 150 µg/ml of ampicillin at 37°C overnight.

Then 10 µl of the culture broth was added to 2 ml of 2YT broth (1.6% bactotrypton, 1% yeast extract, 0.5% NaCl) and 20 µl of a helper phage M13K07 was added thereto. After allowing the mixture to stand at 37°C for 1 hour, kanamycin was added thereto in such a manner as to give a concentration of 70 µg/ml, followed by culturing at 37°C at 150 rpm for 16 hours. 1.5 ml of this culture broth was centrifuged at 10,000 rpm for 10 minutes and thus 1.3 ml of a culture supernatant was recovered. After centrifuging at 10,000 rpm for additional 10 minutes, 1.2 ml of a culture supernatant was obtained.

To this supernatant was added 300 µl of 20% PEG 6000 2.5M NaCl. After being left to stand at room temperature for 30 minutes, the mixture was centrifuged at 10,000 rpm for 10 minutes and the supernatant was removed. The obtained precipitate was dissolved by adding 100 µl of the TE buffer solution thereto and extracted with phenol. After thus removing proteins, the DNA was precipitated with ethanol. Thus single-stranded plasmid DNAs were obtained respectively from the plasmids pUC-PLC containing the PL7-6 H chain variable region gene inserted therein and pUC-1.9K.

By using these single-stranded plasmid DNAs and the double-stranded plasmid DNAs corresponding thereto, which had been prepared in Example 1-(3)-A) and Example 1-(4), the DNA sequences of the terminal DNAs of the inserted fragments were analyzed by the dideoxy method. As a result, the DNA sequence of the M4 primer side shown by SEQ ID No. 4 in the Sequence Listing and the DNA sequence of the RV primer side shown by SEQ ID No. 5 therein were obtained regarding the PL7-6 H chain variable region gene. Regarding the probe J_{H}-1.9K, on the other hand, the DNA sequence of the M4 primer side shown by SEQ ID No. 6 in the Sequence Listing was obtained. Thus it was confirmed that the sequence of the RV primer of the PL7-6 H chain variable region gene was identical with a portion of the DNA sequence of the M4 primer side of the probe J_{H}-1.9K.

### (5) Construction of pSV2HG1-PLH involving PL7-6 H chain variable region gene and human IgG γ-type H chain constant region gene

Examples of plasmid vectors containing a gene coding for the human IgG γ-type H chain constant region integrated thereinto involve pSV2HG1gpt as described in the above-mentioned FEBS Letters. 5 µg of this pSV2HG1gpt was digested with 30 units of EcoRI in 100 µl of a reaction mixture containing the H buffer solution for restriction enzyme reaction at 37°C overnight. Then 0.9 units of an alkaline phosphatase of E. coli was added to this digested reaction mixture and reacted at 65°C for 1 hour. After removing proteins by extraction with phenol, the DNA was precipitated with ethanol and dissolved in 10 µl of the TE buffer solution.

58 µg of the plasmid pUC-PLH was digested with 100 units of EcoRI in 40 µl of a reaction mixture containing the H buffer solution for restriction enzyme reaction at 37°C overnight. Then the reaction mixture was extracted with phenol and DNA was precipitated with ethanol. Thus 28 µg of a DNA mixture comprising the vector pUC119 and the PL7-6 H chain variable region gene was obtained in 100 µl of the TE buffer solution. Next, a DNA fragment of approximately 3.2 kbp was fractionated. 28 ng of this DNA fragment of approximately 3.2 kbp and 1 µg of pSV2HG1gpt including the human IgG γ-type H chain constant region gene integrated therein, which had been digested with EcoRI and dephosphorylated as described above, were reacted at 16°C overnight with the use of a DNA Ligation Kit (Takara Shuzo) in accordance with the protocol of the kit. Thus a objective plasmid including a gene coding for the H chain variable region was obtained. Then E. coli JM109 was transformed with this plasmid and the obtained transformant was applied onto an L-agar medium plate, which contained 100 µg/ml of ampicillin and was coated with 50 µl of 20 mg/ml X-gal and 25 µl of 100 mM IPTG, and white colonies grown on the plate were cultured in L-broth at 37°C overnight. After the completion of the culture, a plasmid was extracted from the cells. Part of the plasmid thus obtained was digested with restriction enzymes EcoRI (5 units) and HincII (5 units) in 15 µl of a reaction mixture containing the H buffer solution for restriction enzyme reaction at 37°C overnight. This reaction mixture was then subjected to electrophoresis on a 1% agarose gel. Thus bands originating from pSV2HG1gpt (6.0 kbp, 4.0 kbp and 0.5 kbp) and those originating from the gene coding for the H chain variable region (2.5 kbp and 0.7 kbp) were confirmed.

Subsequently, the DNA sequence was analyzed by the dideoxy method in order to determine the direction of the inserted DNA fragment of 3.2 kbp in the plasmid. Namely, the double-stranded DNA of the above-mentioned target plasmid was sequenced with the use of a pBR322 Primer R (Takara Shuzo) in accordance with the protocol of Sequenase verII (United States Biochemical). When compared with the DNA sequence of the pUC-PLH analyzed in the above Example 1-(4), the DNA sequence on the Primer R side agreed with that on the M4 Primer side of the pUC-PLH. Thus the target DNA and plasmid including a gene coding for the PL7-6 H chain variable region and another gene coding for the human IgG γ-type H chain constant region integrated thereinto and capable of expressing a chimeric PL7-6 H chain were obtained. This plasmid was named pSV2HG1-PLH. Figure 2 shows a process for constructing this plasmid.

### (6) Identification and isolation of recombinant phage φE4PLL including PL7-6 mouse antibody L chain variable region gene

By using the PL7-6 genomic DNA library employed in the above Example 1-(3), screening was performed in accordance with the procedure of Example 1-(3) with the use of a DNA probe prepared by the following method.

Hybridoma PL7-6 (FERM P-11073) was cultivated and RNA was prepared from the cell. And then, mRNA was prepared from the RNA by the use of Oligotex™-dT30 (Takara Shuzo). Next, cDNA was synthesized from above-mentioned mRNA by the use of reverse transcriptase and primer C-1, specific for constant region of L chain, shown by SEQ ID No. 14 in the Sequence Listing. And then, DNA coding for variable region of L chain was obtained by carrying out 1st-PCR with the primer C-1 and mixed primer VL shown by SEQ ID No. 15 in the Sequence Listing. Next,the DNA coding for variable region of L chain was obtained by carrying out 2nd PCR with the amplified DNA as template and mixed primer VL and specific primer C-2 shown by SEQ ID No. 16 in the Sequence Listing. Southern blot analysis of the amplified DNA was performed with probe LP shown by SEQ ID No. 17 in the Sequence Listing. Thus it was confirmed that target DNA was amplified. The amplified DNA was subjected to electrophoresis on agarose gel and then purified by eluting from the gel. And then, the DNA was subcloned into the Hinc II site of pUC18 after blunting its ends of the DNA with DNA Blunting kit (Takara Shuzo). DNA sequence of the subcloned DNA was analyzed by the dideoxy method. The DNA sequence was shown by SEQ ID No. 7 in the Sequence Listing.

2 µg of this plasmid was digested with 20 units of EcoO109I in 50 µl of a reaction mixture containing the L buffer solution for restriction enzyme reaction (100 mM Tris-HCl, pH 7.5, 100 Mm MgCl₂, 10 mM dithiothreitol) at 37°C overnight. After heating the reaction mixture at 70°C for 15 minutes and precipitating with ethanol, the obtained DNA was dissolved in 8 µl of sterilized water. Then the ends of the DNA fragment were blunted by treating the sample solution with a Blunting Kit (Takara Shuzo) and then heated at 70°C for 15 minutes to thereby inactivate the enzyme. Next, 43 µl of this reaction mixture was treated with 20 units of HindIII in 50 µl of a reaction mixture containing the M buffer solution for restriction enzyme reaction (100 mM Tris-HCl, pH 7.5, 100 mM MgCl₂, 10 mM dithiothreitol, 500 mM NaCl) at 37°C overnight. Then the reaction mixture was subjected to electrophoresis on a 1% agarose gel. A slice of agarose gel containing a fragment of approximately 300 bp was cut out from the gel and the fragment was purified. After precipitating with ethanol, the obtained sample was dissolved in 4 µl of the TE buffer solution.

Next, 4 µg of pUC119 was digested with 10 units of SmaI in 25 µl of a reaction mixture containing the L buffer solution for restriction enzyme reaction at 37°C overnight. After being heated at 70°C for 15 minutes, 25 µl of the sample solution was digested with 20 units of HindIII in 100 µl of a reaction mixture containing the M buffer solution for restriction enzyme reaction at 37°C overnight. Subsequently, 1 units of an alkaline phosphatase originating from E. coli was added to this reaction mixture, followed by a reaction at 65°C for 1 hour. After the completion of the reaction, proteins were removed from the reaction mixture by extraction with phenol. Then the DNA was precipitated with ethanol and the sample thus obtained was dissolved in 25 µl of the TE buffer solution.

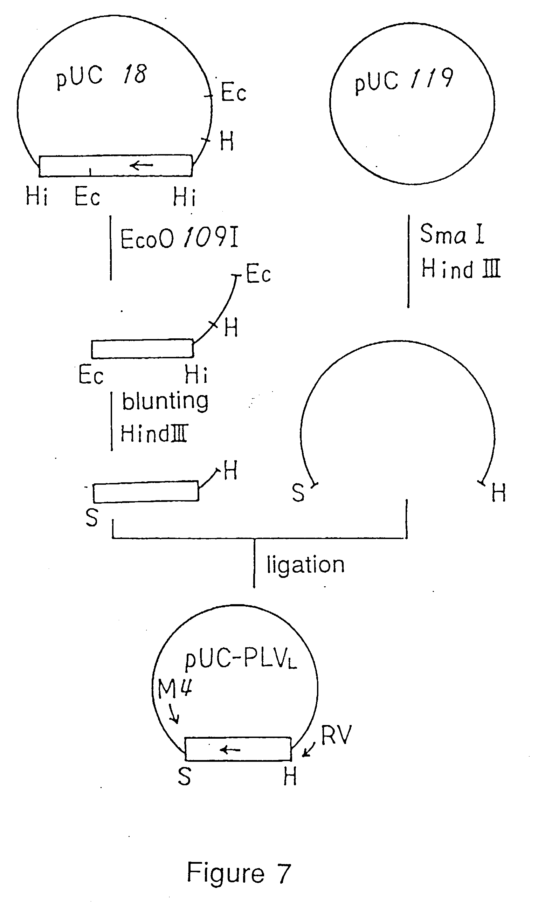

This pUC119, which had been digested with SmaI and HindIII and dephosphorylated, and the DNA fragment of approximately 300 bp prepared above were reacted at 16°C overnight with the use of a Ligation Kit (Takara Shuzo). Thus a plasmid into which this fragment of approximately 300 bp had been integrated was obtained and named pUC-PLV_{L}. Figure 7 shows a process for constructing this plasmid, wherein Ec means EcoO109I, Hi means HincII and S means SmaI.

Subsequently, a DNA fragment on pUC-PLV_{L}, shown by SEQ ID No. 8 in the Sequence Listing, containing a DNA sequence coding for the L chain variable region of PL7-6 was amplified by the PCR method in accordance with the above Example 1-(3)-A). Thus approximately 1 µg of the DNA fragment was obtained from 100 µl of the PCR reaction mixture. This DNA was named probe PL-V_{L} for use in the subsequent steps.

The sequence ranging from the base No. 51 to the base No. 242 in SEQ ID No. 8 in the Sequence Listing corresponds to the portion of the DNA sequence coding for the PL7-6 antibody L chain variable region.

The probe PL-V_{L} was subjected to nonradioactive labeling with the use of a Chemi Probe Kit (Takara Shuzo) to conduct the primary screening in accordance with the procedure described in the above Example 1-(3). Thus 4 positive clones were obtained. In the subsequent secondary screening, the presence of 2 positive clones were confirmed. Then a phage lysate was prepared in accordance substantially with the method described in Experimental Medicine (Jikken Igaku), 5(11), 28-32 (1987) and then a phage DNA was prepared.

Approximately 5 µg of this phage DNA was completely digested by incubating together with 10 units of EcoRI, 10 units of BamHI and 0.25 µg of RNaseA in 15 µl of a reaction mixture containing the H buffer solution for restriction enzyme reaction at 37°C for 6 hours. This reaction mixture was then subjected to electrophoresis on a 0.7% agarose gel and DNA fragments were transferred onto a nylon membrane Hybond-N (Amersham). After Southern hybridization with the probe PL-V_{L}, a DNA fragment of approximately 7.0 kbp capable of hybridizing with the probe PL-V_{L} was confirmed.

A phage clone containing this DNA fragment of approximately 7.0 kbp was named φE4PLL.

### (7) Subcloning of phage DNA fragment including PL7-6 mouse antibody L chain variable region gene

The above-mentioned φE4PLL DNA was isolated and approximately 5 µg thereof was completely digested by incubating together with 10 units of EcoRI, 10 units of BamHI and 0.5 µg of RNaseA in 30 µl of a reaction mixture containing the H buffer solution for restriction enzyme reaction at 37°C overnight.

After subjecting the DNA thus treated to electrophoresis on a 0.7% agarose gel, a slice of agarose gel containing a fragment of approximately 7.0 kbp was cut out and the fragment was purified. Then it was subcloned into the EcoRI and BamHI sites of pUC119 in the same manner as the one described in the above Example 1-(4). The recombinant plasmid thus obtained was named pUC-7.0.

Next, E. coli JM109 was transformed with this pUC-7.0 in accordance with the method of the above Example 1-(4). After transfecting the obtained transformant with a helper phage M13K07, the culture supernatant was prepared. Thus a single-stranded plasmid DNA for the plasmid pUC-7.0 involving the PL7-6 L chain variable region gene inserted thereinto was obtained.

By using this single-stranded plasmid and the above-mentioned double-stranded DNA corresponding thereto, the DNA sequences of the terminals of the inserted fragment were analyzed by the dideoxy method. Thus the partial DNA sequence of the fragment on the M4 primer side shown by SEQ ID No. 9 in the Sequence Listing and that on the RV primer side shown by SEQ ID No. 10 in the Sequence Listing were determined. As the result of the searching with the use of a DNASIS™ Gene/Protein Sequence Data Base (Hitachi Software Engineering), the DNA sequence of the fragment on the RV primer side (SEQ ID No. 10) agreed with the sequence on the downstream side of the mouse antibody κ-chain constant region. Thus, it was found out that the DNA coding for the mouse κ-chain constant region was included in the fragment inserted into the plasmid pUC-7.0.

### (8) Elimination of gene coding for mouse κ-chain constant region from pUC-7.0

An EcoRI, HindIII fragment of the plasmid pUC-7.0 was prepared and the target DNA fragment of approximately 4 kbp, in which the gene coding for the κ-chain constant region had not been contained, was named PLL. The restriction enzyme map of this DNA fragment was prepared. Figure 3 shows the restriction enzyme map thus obtained. This fragment had neither the recognition site of BamHI nor that of SphI. Next, this PLL was subjected to linker-ligation with a phosphorylated HindIII linker (Takara Shuzo) and cloned again into the HindIII site of the vector pUC119.

The plasmid thus obtained was named pUC-PLL.

### (9) Construction of plasmid pSV2HCκ-PLL including PL7-6 L chain variable region gene and human IgG κ-type L chain constant region gene

Examples of plasmid vectors containing a gene coding for the human IgG κ-type L chain constant region include pSV2-HCκneo as described in the above-mentioned FEBS Letters. A DNA fragment of approximately 4.0 kbp involving the PL7-6 L chain variable region gene, which had been obtained from this plasmid pUC-PLL as a HindIII digestion fragment, was cloned into the Hind III site of the plasmid pSV2-HCκneo in accordance with the method of the above Example 1-(5).

Next, the direction of the DNA fragment of approximately 4 kbp thus inserted was determined by analyzing the DNA sequence by the dideoxy method in accordance with the method of the above Example 1-(4). When the result thus obtained was compared with the result of the analysis of the above Example 1-(7), the DNA sequence of this fragment agreed with the above-mentioned DNA sequence on the M4 primer side of the PL7-6 L chain variable region gene shown by SEQ ID No. 9. Thus a plasmid, including the gene coding for the PL7-6 L chain variable region and the gene coding for the human IgG κ-type L chain constant region and capable of expressing the chimeric PL7-6 L chain, were obtained. This plasmid was named pSV2HCκ-PLL. Figure 4 shows a process for constructing this plasmid.

### Example 2

### (1) Introduction of plasmids pSV2HG1-PLH and pSV2HCκ-PLL into myeloma cells

Mouse myeloma cells SP2/0 were well grown in a medium RPMI1640 containing 10% fetal calf serum (FCS), 50 units/ml penicillin and 50 µg/ml streptomycin (hereinafter referred to as the basal medium) so as to give a density of 6 x 10⁴ cells/ml of the basal medium. 8 ml of the culture medium was taken up and centrifuged at room temperature at 1,000 rpm for 30 minutes. Then the supernatant was removed and thus the cells were collected.

Next, 100 µl of 1 mg/ml DOTMA [N-{1-(2,3-dioleyloxy)propyl)-N,N,N-trimethylammonium chloride; Boehringer-Mannheim], 10 µg of pSV2HG1-PLH and 10 µg of pSV2HCκ-PLL were diluted with 8 ml of the basal medium and added to the cells collected above and incubated at 37°C for 6 hours. After centrifuging at room temperature at 1,000 rpm for 30 seconds, the cells were collected again. Then 8 ml of the fresh basal medium was added thereto to continue incubation at 37°C for 48 hours.

Subsequently, the basal medium was replaced with a selection medium containing 400 µg/ml of Geneticin (Sigma) and transferred into a 96-well culture plate to perform incubation at 37°C.

### (2) Selection of positive clones

In accordance with the method described in Japanese Patent Laid-Open No. 22866/1992, human GMP-140 was solubilized from human platelets with the use of a detergent and purified. Then 5 µg/ml solution of this purified human GMP-140 in PBS was added to a 96-well ELISA plastic plate at a ratio of 50 µl per well to cause adsorption at 4°C overnight. Then the solution was removed and 200 µl/well of a 1% solution of bovine serum albumin in PBS was added thereto, followed by incubating at 4°C overnight. After the completion of the blocking, the plate was washed with a PBS solution thrice and 50 µl/well of the culture supernatant was added thereto. After conducting a reaction at 37°C for an hour, the plate was washed with the PBS solution thrice. Then 100 µl/well of the solution of horseradish peroxidase-labeled anti-human IgG antibody (Cappel) in PBS was added thereto and reacted at 37°C for an hour. After the completion of the reaction, the plate was washed with PBS 4 times. Next, 100 µl portions of a citrate buffer solution (pH 5.0) containing 0.01% hydrogen peroxide and 2 mM ABTS [2,2'-adinobis(3-ethylbenzothiazoline-6-sulfonic acid) diammonium salt] were added to the washed plate and reacted at room temperature for 20 minutes. Then the reaction was ceased by adding 100 µl of 0.15 M oxalic acid. The absorbance at 405 nm was measured and thus positive clones were selected.

These antibody-producing strains were cloned by the limiting dilution method. Thus a clone cPL-2R1 strain of SP2/0 transformant capable of producing an antibody of the highest titer was obtained.

### (3) Collection of antibody

The above-mentioned cPL-2R1 strain was cultured in a serum-free medium of RPMI1640 to thereby give 360 ml of a culture supernatant. This supernatant was purified by salting out with ammonium sulfate and DEAE cellulose chromatography in the conventional manner. Thus approximately 50 µg of the target antibody was obtained in a purified state. Then the antigen recognizing ability of this antibody was confirmed. This antibody was named chimeric PL7-6 while the strain producing this antibody was named Myeloma SP2-cPL-2R1 and deposited with Fermentation Research Institute of the Agency of Industrial Science and Technology as FERM BP-3609.

### Example 3

### (1) Immunoblotting analysis for antibody structure

10 µg each of PL7-6 (Takara Shuzo), chimeric PL7-6, or human IgG (Cappel-Organon Teknika) was reduced by heated for 5 min at 100°C with β-mercaptoethanol, and separated on SDS-polyacrylamide slab electrophoresis using 15% gel. After electrophoresis, the proteins were electrophoretically transferred onto polyvinylidene difluoride (PVDF) membrane (Millipore). One of these membranes was subjected to the protein-staining procedure. The other sheets were blocked by incubated with phosphate-buffered saline (PBS) containing 1% skim milk (Difco Lab.), and futher incubated with PBS solution contianing peroxidase-labelled anti-mouse IgG whole molecule (Cappel) or peroxidase-labelled anti-human IgG Fc region (Cappel). Immunoreactive proteins on the membranes were visualized by color intensity of 4-chloro-1-naphtol substrate (Sigma).

The data obtained by the above analysis described that PL7-6, chimeric PL7-6, and human IgG were constructed with IgG heavy chain having molecular mass of 55 kDa and IgG light chain having molecular mass of 28 kDa, and that enzyme-labelled anti-mouse IgG whole molecule was shown to react only with a small amount of chimeric PL7-6 heavy chain and PL7-6 IgG (heavy and light chain), and that enzyme-labelled anti-human IgG Fc region was shown to react only with heavy chain of chimeric PL7-6 and human IgG. These results demonstrated that the chimeric PL7-6 antibody had a human IgG-like heavy chain and was containing a little slight amount of mouse IgG-like structure.

### (2) Reactivities of antibodies with platelet antigen

PL52-4 monoclonal antibody (Takara Shuzo) was used as an anti-human platelet glycoprotein Ib antibody. Washed platelets were prepared from human blood according to the published method by Timmons and Hawiger [Methods in Enzymology, 169, 11-21, (1989)], and separated on a SDS-polyacrylamide gel electrophoresis using 7.5% gel under reducing with β-mercaptoethanol or non-reducing condition. After electrophoresis, the proteins in the gel were immediately electrophoretically transferred onto the PVDF membranes according to the standard western blotting technology. Transblotted proteins on these PVDF membranes were stained with Coomassie Brilliant Blue G-250 dye (Nacalai tesque), or blocked by PBS containing 1% skim milk and immunostained with peroxidase-labelled rabbit antibodies specific to mouse IgG whole molecule or human IgG-Fc region and 4-chloro-1-naphthol substrate (Sigma). Immunoreactive antigen on the membranes was detected by enzymatic color intensity of substrate. GMP-140 antigen, whose apparent molecular mass is found to be about 135 kDa on a SDS-polyacrylamide gel electrophoresis without reduction, was shown to be recognized by PL7-6 and chimeric PL7-6 antibodies under non-reducing condition only, and none was detected by both of these antibodies in the platelet proteins separated on a SDS-polyacrylamide gel electrophoresis under reducing condition. In the same electrophoretic run, glycoprotein Ib antigen on another membrane could be detected to be about 127 kDa by using PL52-4 antibody under reducing and nonreducing conditions.

These results demonstrated that specificity of chimeric PL7-6 antibody was identical to that of PL7-6.

### Example 4

### (1) Characterization of antibody by competitive enzyme-linked immunosorbent assay

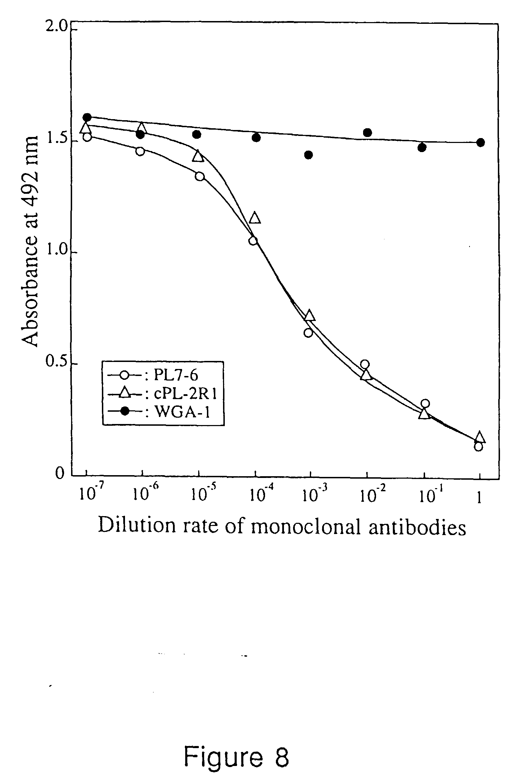

Competitive immunoassay of PL7-6 and chimeric PL7-6 antibodies for purified GMP-140 was performed as follows. First, GMP-140 was purified from out-dated platelet concentrates and purified PL7-6 MoAbs were labelled with horseradish peroxidase. Then 100µl each of PBS solution containing 1 µg/ml purified GMP-140 was added into the well of the microtiter plate (Nunc) and incubated overnight at 4°C. The wells were blocked by PBS containing 1% skim milk after the purified antigen was immobilized on the microtiter plate. PL7-6, chimeric PL76, and WGA-l (Takara Shuzo) antibodies were added at several concentrations (500 µg/ml at maximum) and incubated for 1 hour at 37°C. Peroxidase-labelled PL7-6 antibodies were added after the microplate was washed twice by PBS and allowed to react for 1 hour at 37°C. After incubation, the plates were washed twice with PBS and bound PL7-6 conjugates were estimated by optically measuring enzymatic color intensity of o-phenylenediamine-2HCl substrate (Sigma) at 492 nm using a Titertek multiscan platereader (Flow Laboratories). Competitive inhibition of enzyme-labelled PL7-6 binding to GMP-140 immobilized on the microplate by the pretreatment of these antibodies was shown in Fig. 8.

### (2) Flowcytometric analysis of antibody

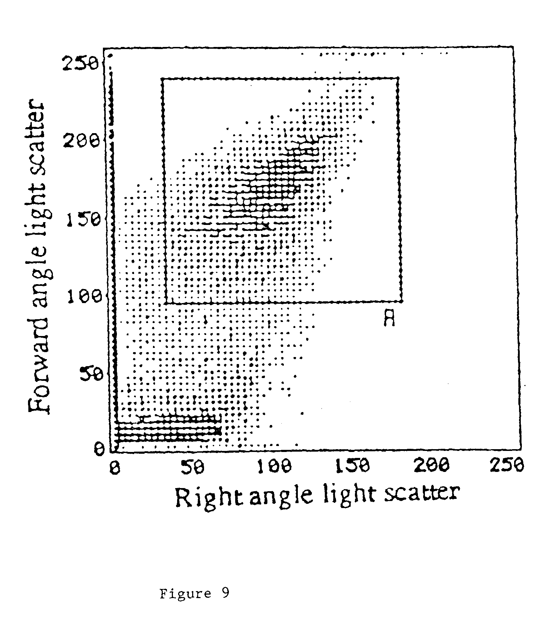

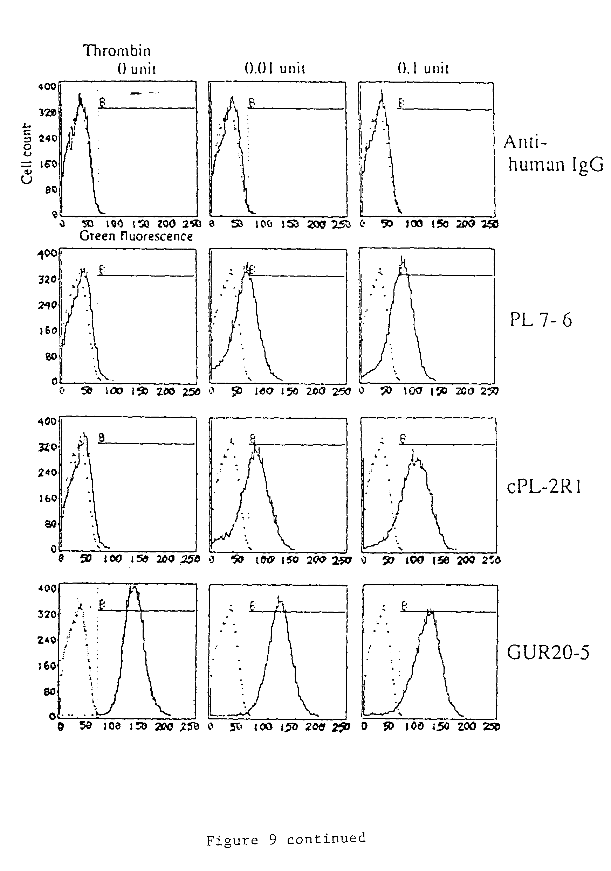

PL7-6, chimeric PL7-6, and GUR20-5 (anti-human platelet glycoprotein Ib antibody described in Int. J. Hematol. 55 Supplement No.1 253 (1992)) antibodies were labelled with FITC isomer-1 (Dojindo Lab.). Human washed platelets were prepared from fresh drawn blood as described in experiment 3-(2). 0, 0.01, and 0.1 units of bovine plasma thrombin (Sigma) was added to fresh washed platelets and the mixtures were incubated for 5 minutes. Then, they were fixed in 0.5% paraformaldehyde (Wako Pure Chemical) for 5 minutes and washed twice with PBS. The fixed platelets were incubated in PBS containing 1 µg/ml FITC-conjugated anti-human IgG, PL7-6, chimeric PL7-6, and GUR20-5, for 30 minutes at 4°C. In the flowcytometric analysis, platelets were nestgated in the first threshold of the forward and right angle light scatters, and secondly, their fluorescence activities on the surfaces of fixed platelets were measured in the Cytoron analyzer (Ortho Diagnostic Systems). Particle population gated in the forward and right angle light scatters, and fluorescence intensities on platelet surfaces treated with thrombin at several concentrations were shown in Fig. 9.

These data described that PL7-6 and chimeric PL7-6 antibodies were more reactive with thrombin-stimulated platelets and less reactive with non-stimulated platelets, and that reactivities of the other antibodies with platelets were not affected with thrombinstimulation. These results demonstrated that chimeric PL7-6 was specifically reactive to thrombin-stimulated platelets as well as PL7-6.

### [Effects of the Invention]

According to the present invention, a highly safe chimeric antibody which can recognize human GMP-140 and is useful in the fields of drugs and diagnosis can be provided. Furthermore, transformed cells capable of producing the above-mentioned antibody are produced by the genetic engineering technology and thus a means for producing the chimeric antibody on an industrial scale is provided.

### SEQUENCE LISTING

SEQ ID NO:1
   LENGTH:351 base pairs
   TYPE:nucleic acid
   STRANDEDNESS:double
   TOPOLOGY:linear
   MOLECULE TYPE:cDNA to mRNA
SEQUENCE DESCRIPTION: ID NO:1: 
GAGGTCCAGC TCCAGCAGTC CGGAGCCGAA CTGACAAAAC CTGGGGCCTC AGTGAATATG
60
TCCTGCAAGG CTTCTGGCTA CACCTTTACT AGCTACTGGA TGCACTGGGT AAAACAGAGG
120
CCTGGACAGG GTCTGGAATG GATTGGATAC ATTAATCCTG GCACTGCTTA TACTGAGCAC
180
AATCAGAAGT TCAAGGACAA GGCCACATTG ACTGCAGACA AGTCCTCCAG CACAGCCTAC
240
ATGCAACTGA GCAGCCTGAC ATCTGAGGAC TCTGCAGTCT ATTACTGTGC AAGTGGTAAC
300
CCCGCCTGGT TTGCTTACTG GGGCCAAGGG ACTCTGGTCA CTGTCTCTGC A
351
SEQ ID NO:2
   LENGTH:216 base pairs
   TYPE:nucleic acid
   STRANDEDNESS:double
   TOPOLOGY:linear
   MOLECULE TYPE:cDNA to mRNA
SEQUENCE DESCRIPTION: ID NO:2: 
GAGGTCCAGC TCCAGCAGTC CGGAGCCGAA CTGACAAAAC CTGGGGCCTC AGTGAATATG
60
TCCTGCAAGG CTTCTGGCTA CACCTTTACT AGCTACTGGA TGCACTGGGT AAAACAGAGG
120
CCTGGACAGG GTCTGGAATG GATTGGATAC ATTAATCCTG GCACTGCTTA TACTGAGCAC
180
 AATCAGAAGT TCAAGGACAA GGCCACATTG ACTGCA
SEQ ID NO:3
   LENGTH:331 base pairs
   TYPE:nucleic acid
   STRANDEDNESS:double
   TOPOLOGY:linear
SEQUENCE DESCRIPTION: ID NO:3: 
CAGGAAACAG CTATGACCAT GATTACGAAT TCGAGCTCGG TACCCGGGGA TTCTCTAGAG
60
TCGAGGTCCA GCTCCAGCAG TCCGGAGCCG AACTGACAAA ACCTGGGGCC TCAGTGAATA
120
TGTCCTGCAA GGCTTCTGGC TACACCTTTA CTAGCTACTG GATGCACTGG GTAAAACAGA
180
GGCCTGGACA GGGTCTGGAA TGGATTGGAT ACATTAATCC TGGCACTGCT TATACTGAGC
240
ACAATCAGAA GTTCAAGGAC AAGGCCACAT TGACTGCAGG CATGCAAGCT TGGCACTGGC
300
CGTCGTTTTA CAACGTCGTG ACTGGGAAAA C
331
SEQ ID NO:4
   LENGTH:272 base pairs
   TYPE:nucleic acid
   STRANDEDNESS:double
   TOPOLOGY:linear
SEQUENCE DESCRIPTION: ID NO:4: 
ATGCAGGACC AGAGATTGCC CTGTAAACTA TCAGACTTCA AGTAAGATGA TTTCCCTAGT
60
GTCTGATTGT TACATAGTGA CCAATTACTT CCTAATTCCT AATCCATATG AAATATAGAA
120
ATAATAATTT TACATATATT TAGAATTTTC ATAAAATGTA TTTTGTAAAT GGAACCTATT
180
TCTAACATAA CTTCTACCCT TCCTTTGTTG CCCACATATT CTCTAACTAT TCAACTAAAT
240
272
TTCACTGAAA TATCATGTAT TTCATACATA TG
SEQ ID NO:5
   LENGTH:66 base pairs
   TYPE:nucleic acid
   STRANDEDNESS:double
   TOPOLOGY:linear
SEQUENCE DESCRIPTION: ID NO:5: 
TAAATACATT TTAGAAGTCG ATAAACTTAA GTTTGGGGAA ACTAGAACTA CTCAAGCTAA
60
AATTAA
66
SEQ ID NO:6
   LENGTH: 186 base pairs
   TYPE:nucleic acid
   STRANDEDNESS:double
   TOPOLOGY:linear
SEQUENCE DESCRIPTION: ID NO:6: 
TAAATACATT TTAGAAGTCG ATAAACTTAA GTTTGGGGAA ACTAGAACTA CTCAAGCTAA
60
AAGTAAAAGG TTGAACTCAA TAAGTTAAAA GAGGACCTCT CCAGTTTCGG CTGAATCCTC
120
AACTTATTTT AGAAATGCAA ATTACCCAGG TGGTGCCCCG CTCAGCCTGG ACTTTCGGTT
180
TGGTGG 186
SEQ ID NO:7
   LENGTH:339 base pairs
   TYPE:nucleic acid
   STRANDEDNESS:double
   TOPOLOGY:linear
   MOLECULE TYPE:cDNA to mRNA
SEQUENCE DESCRIPTION: ID NO:7: 
GATATTGTCC TTGCTCAATC ACCAGCCTCC CTGGCTATGT CAGTAGGACA GAAGGTCACT
60
ATGAGCTGCA AGTCCAGTCA GAGCCTTTTA AATAGTAGCA ATCAAAAGAA CTATTTGGCC
120
TGGTACCAGC AGAAACCAGG ACAGTCTCCT AAACTTCTGG TATACTTTGC ATCCACTAGG
180
GAATCTGGGG TCCCTGATCG CTTCATAGGC AGTGGATCTG GGACAGATTT CACTCTTACC
240
GTCAGCAGTG TGCAGGCTGA AGACCTAGCA GATTACTTCT GTCAGCAACA TTATAGCACT
300
CCTCGGACGT TCGGTGGAGG CACCAAGCTG GAGATCAAA 339
SEQ ID NO:8
   LENGTH:298 base pairs
   TYPE:nucleic acid
   STRANDEDNESS:double
   TOPOLOGY:linear
SEQUENCE DESCRIPTION: ID NO:8: 
CAGGAAACAG CTATGACCAT GATTACGCCA AGCTTGCATG CCTGCAGGTC GATATTGTCC
60
TTGCTCAATC ACCAGCCTCC CTGGCTATGT CAGTAGGACA GAAGGTCACT ATGAGCTGCA
120
AGTCCAGTCA GAGCCTTTTA AATAGTAGCA ATCAAAAGAA CTATTTGGCC TGGTACCAGC
180
AGAAACCAGG ACAGTCTCCT AAACTTCTGG TATACTTTGC ATCCACTAGG GAATCTGGGG
240
TCGGGTACCG AGCTCGAATT CACTGGCCGT CGTTTTACAA CGTCGTGACT GGGAAAAC
298
SEQ ID NO:9
   LENGTH:215 base pairs
   TYPE:nucleic acid
   STRANDEDNESS:double
   TOPOLOGY:linear
SEQUENCE DESCRIPTION: ID NO:9: 
TCTAAAATAT GCCTTTGTGC AATAGACCCT TTCAGTGTTG CTGGTGTCCA GGCATGATGA
60
GGTAAAGAAA CCTTGTTTAT TTTATTCTCC ACCATTTCTG CAAAAATTAG AACCCCTTGT
120
AAGTTCCCTG AAGTTGTAGG AAACATGTTA CTTTCTGTGG GGTTTGCTTT TACTTTGAGA
180
CTAAATATTC ACTGATCCTA GGCTTAAATT CATGT
215
SEQ ID NO:10
   LENGTH:185 base pairs
   TYPE:nucleic acid
   STRANDEDNESS:double
   TOPOLOGY:linear
SEQUENCE DESCRIPTION: ID NO:10: 
CCTACTTTAT TGTCTTTCTG GCCTTGACGT ATGATTCATT CCTTGTATCC TCTTTTATGT
60
ATGCACTGGG TATTAGATAT TTCTTTAGGT GAAGTTATTA TGAAGTATAC AATATATACA
120
GGAAGAACAT AGATCCCAAT TCCACAATAG TAACTTTCAC ATCCCATACA GAGAGACAGA
180
185
ACATT
SEQ ID NO:11
   LENGTH:14 bases
   TYPE:nucleic acid
   STRANDEDNESS:single
   TOPOLOGY:linear
   MOLECULE TYPE:Other nucleic acid (synthetic DNA)
   ANTI-SENSE:YES
SEQUENCE DESCRIPTION: ID NO:11: GGCCAGTGGA TAGA 14
SEQ ID NO:12
   LENGTH:29 bases
   TYPE:nucleic acid
   STRANDEDNESS:single
   TOPOLOGY:linear
   MOLECULE TYPE:Other nucleic acid (synthetic DNA)
   ANTI-SENSE:NO
SEQUENCE DESCRIPTION: ID NO:12: GARGTVCAGC TVCAGCARTC NGGMGCMGA 29
SEQ ID NO:13
   LENGTH:22
   TYPE:nucleic acid
   STRANDEDNESS:single
   TOPOLOGY:linear
   MOLECULE TYPE:Other nucleic acid (synthetic DNA)
   ANTI-SENSE:NO
SEQUENCE DESCRIPTION: ID NO:13: CHGATGGGGS TGTYGTTTTG GC 22
SEQ ID NO:14
   LENGTH:20 bases
   TYPE:nucleic acid
   STRANDEDNESS:single
   TOPOLOGY:linear
   MOLECULE TYPE:Other nucleic acid (synthetic DNA)
   ANTI-SENSE:YES
SEQUENCE DESCRIPTION: ID NO:14: CCAGATGTTA ACTGCTCACT 20
SEQ ID NO:15
   LENGTH:26 bases
   TYPE:nucleic acid
   STRANDEDNESS:single
   TOPOLOGY:linear
   MOLECULE TYPE:Other nucleic acid (synthetic DNA)
   ANTI-SENSE:NO
SEQUENCE DESCRIPTION: ID NO:15: GAYATYGTSC TBACHCARTC NCCAGC 26
SEQ ID NO:16
   LENGTH:14 bases
   TYPE:nucleic acid
   STRANDEDNESS:single
   TOPOLOGY:linear
   MOLECULE TYPE:Other nucleic acid (synthetic DNA)
   ANTI-SENSE:YES
SEQUENCE DESCRIPTION: ID NO:16: TGGTGGGAAG ATGG 14
SEQ ID NO:17
   LENGTH:20 bases
   TYPE:nucleic acid
   STRANDEDNESS:single
   TOPOLOGY:linear
   MOLECULE TYPE:Other nucleic acid (synthetic DNA)
   ANTI-SENSE:NO
SEQUENCE DESCRIPTION: ID NO:17:

TACAGTTGGT GCAGCATCAG 20

## Claims

1. An isolated DNA fragment encoding a mouse antibody variable region and a human antibody constant region of a chimeric antibody which binds human GMP-140 specifically and having:
(i) the nucleotide sequence shown by SEQ. ID NO: 1 encoding the mouse H chain variable region or SEQ ID NO:7 encoding the mouse L chain variable region;
(ii) a sequence corresponding to the sequence of (i) within the scope of the degeneracy of the genetic code; or
(iii a sequence which hybridizes with a sequence complementary to the sequence (i) or (ii) under stringent conditions.

2. A method for producing a chimeric antibody which binds human GMP-140 specifically comprising the following steps:
(a) transforming mammalian cells with a vector containing a mouse H chain variable region gene of claim 1 and a human H chain constant region gene, and another vector containing a mouse L chain variable region gene of claim 1 and a human L chain constant region gene,
(b) isolating the said chimeric antibody from a culture medium in which the transformed cells have been cultivated.

3. A method of claim 2, wherein the mouse H chain variable region gene is represented by the restriction map shown in Figure 1.

4. A method of claim 2, wherein the mouse L chain variable region gene is represented by the restriction map shown in Figure 3.

5. A transformed cell which has been transformed with a vector containing a mouse H chain variable region gene of claim 1 and a human H chain constant region gene, and another vector containing a mouse L chain variable region gene of claim 1 and a human L chain constant region gene, and is capable of producing a chimeric antibody which binds human GMP-140 specifically.

6. A transformed cell of claim 5, wherein the cell is Myeloma SP2-cPL-2R1 (FERM BP-3609).

## Patentansprüche

1. Isoliertes DNA-Fragment, codierend einen variablen Antikörper-Abschnitt aus Maus und einen konstanten Antikörper-Abschnitt aus Mensch eines chimären Antikörpers, der menschliches GMP-140 spezifisch bindet, mit:
(i) der Nukleotidsequenz, gezeigt in SEQ.-ID.-Nr. 1, die den variablen Abschnitt einer H-Kette aus Maus codiert, oder in SEQ.-ID.-Nr. 7, die den variablen Abschnitt einer L-Kette aus Maus codiert;
(ii) einer Sequenz, die der Sequenz aus (i) innerhalb des Bereichs der Degeneration des genetischen Codes entspricht; oder
(iii) einer Sequenz, die unter stringenten Bedingungen mit einer zur Sequenz (i) oder (ii) komplementären Sequenz hybridisiert.

2. Verfahren zur Herstellung eines chimären Antikörpers, der menschliches GMP-140 spezifisch bindet, umfassend die folgenden Schritte:
(a) das Transformieren von Säugetierzellen mit einem Vektor, der ein Gen für einen variablen Abschnitt einer H-Kette aus Maus nach Anspruch 1 und ein Gen für einen konstanten Abschnitt einer H-Kette aus Mensch enthält, und einem weiteren Vektor, der ein Gen für einen variablen Abschnitt einer L-Kette aus Maus nach Anspruch 1 und ein Gen für einen konstanten Abschnitt einer L-Kette aus Mensch enthält,
(b) das Isolieren dieses chimären Antikörpers aus einem Kulturmedium, in dem die transformierten Zellen gezüchtet worden sind.

3. Verfahren nach Anspruch 2, wobei das Gen für den variablen Abschnitt einer H-Kette aus Maus durch die in Figur 1 gezeigte Restriktionskarte veranschaulicht ist.

4. Verfahren nach Anspruch 2, wobei das Gen für den variablen Abschnitt einer L-Kette aus Maus durch die in Figur 3 gezeigte Restriktionskarte veranschaulicht ist.

5. Transformierte Zelle, die mit einem Vektor, der ein Gen für einen variablen Abschnitt einer H-Kette aus Maus nach Anspruch 1 und ein Gen für einen konstanten Abschnitt einer H-Kette aus Mensch enthält, und einem weiteren Vektor transformiert worden ist, der ein Gen für einen variablen Abschnitt einer L-Kette aus Maus nach Anspruch 1 und ein Gen für einen konstanten Abschnitt einer L-Kette aus Mensch enthält, und die einen chimären Antikörper erzeugen kann, der menschliches GMP-140 spezifisch bindet.

6. Transformierte Zelle nach Anspruch 5, wobei die Zelle das Myelom SP2-cPL-2R1 (FERM BP-3609) ist.

## Revendications

1. Fragment d'ADN isolé codant une région variable d'anticorps murin et une région constante d'anticorps humain d'un anticorps chimère qui se lie spécifiquement au GMP-140 humain, et possédant:
(i) la séquence de nucléotides représentée par la séquence identifiée par le n° 1 codant la région murine variable à chaînes H ou par la séquence identifiée par le n° 7 codant la région murine variable à chaînes L;
(ii) une séquence correspondant à la séquence de (i) dans le cadre de la dégénérescence du code génétique; ou
(iii) une séquence qui s'hybride avec une séquence complémentaire de la séquence (i) ou (ii) dans des conditions stringentes.

2. Procédé de production d'un anticorps chimère qui se lie spécifiquement au GMP-140 humain, comprenant les étapes suivantes, qui consistent:
(a) à transformer des cellules de mammifère avec un vecteur contenant un gène murin de région variable à chaînes H suivant la revendication 1 et un gène humain de région constante à chaînes H, et un autre vecteur contenant un gène murin de région variable à chaînes L suivant la revendication 1 et un gène humain de région constante à chaînes L,
(b) à isoler cet anticorps chimère d'un milieu de culture dans lequel les cellules transformées ont été cultivées.

3. Procédé suivant la revendication 2, dans lequel le gène murin de la région variable à chaînes H est représenté par la carte de restriction reproduite sur la figure 1.

4. Procédé suivant la revendication 2, dans lequel le gène murin de la région variable à chaînes L est représenté par la carte de restriction reproduite sur la figure 3.

5. Cellule transformée, dont la transformation a été effectuée avec un vecteur contenant un gène murin de région variable à chaînes H suivant la revendication 1 et un gène humain de région constante à chaînes H, et un autre vecteur contenant un gène murin de région variable à chaînes L suivant la revendication 1 et un gène humain de région constante à chaînes L et qui est capable de produire un anticorps chimère qui se lie spécifiquement au GMP-140 humain.

6. Cellule transformée suivant la revendication 5, cellule qui est le Myélome SP2-cPL-2R1 (FERM BP-3609).
